# EUROPEAN PATENT SPECIFICATION

(11) **EP 2 099 733 B1**
(45) Date of publication and mention of the grant of the patent: **27.03.2013**
(21) Application number: 07867853.9
(22) Date of filing: 20.12.2007
(51) Int. Cl.: C07C 17/20, C07C 17/25, C07C 19/10, C07C 17/354, C07C 21/18

(54) **PROCESS FOR THE SYNTHESIS AND SEPARATION OF HYDROFLUOROOLEFINS**
VERFAHREN ZUR SYNTHESE UND TRENNUNG VON HYDROFLUOROLEFINEN
PROCÉDÉ DE SYNTHÈSE ET DE SÉPARATION D'HYDROFLUORO-OLÉFINES

(30) Priority: 20.12.2006 US 875930 P; 20.12.2006 US 875931 P
(43) Date of publication of application: 16.09.2009
(73) Proprietor: E. I. Du Pont de Nemours and Company, Wilmington, DE 19898 (US)
(72) Inventor: RAO, Velliyur Nott Mallikarjuna, Wilmington, Delaware 19809 (US); NAPPA, Mario Joseph, Newark, Delaware 19711 (US); SIEVERT, Allen Capron, Elkton, Maryland 21921 (US); KNAPP, Jeffrey P., Wilmington, Delaware 19808 (US)
(74) Representative: Matthews, Derek Peter
(86) International application number: PCT/US2007/026001
(87) International publication number: WO 2008/079265

(56) References cited:
- DE-B- 1 139 831
- HENNE A L ET AL: "FLUORINATED DERIVATIVES OF PRAPANE AND PROPYLENE" JOURNAL OF THE AMERICAN CHEMICAL SOCIETY, AMERICAN CHEMICAL SOCIETY, WASHINGTON, DC, US, vol. 68, 1 January 1946 (1946-01-01), pages 496-497, XP002322185 ISSN: 0002-7863

## Description

### CROSS REFERENCE(S) TO RELATED APPLICATION(S)

This application claims the benefit of priority of U.S. Provisional Application 60/875,930, filed December 20, 2006 and U.S. Provisional Application 60/875,931, filed December 20, 2006.

### BACKGROUND INFORMATION

### Field of the Disclosure

This disclosure relates in general to the synthesis of hydrofluoroolefins.

### Description of the Related Art

The refrigeration industry has been working for the past few decades to find replacement refrigerants for the ozone depleting chlorofluorocarbons (CFCs) and hydrochlorofluorocarbons (HCFCs) being phased out as a result of the Montreal Protocol. The solution for most refrigerant producers has been the commercialization of hydrofluorocarbon (HFC) refrigerants. The new HFC refrigerants, HFC-134a being the most widely used at this time, have zero ozone depletion potentials and thus are not affected by the current regulatory phase-out as a result of the Montreal Protocol.

In addition to ozone depleting concerns, global warming is another environmental concern. Thus, there is a need for heat transfer compositions that meet both low ozone depletion as well having low global warming potentials. Certain hydrofluoroolefins meet both goals. Thus there is a need for manufacturing processes that provide halogenated hydrocarbons and fluoroolefins that contain no chlorine that also have a lower global warming potential.

J. CHEM. AM. SOC. 68, 1946, 486-497 discloses fluorinated derivatives of propane and propylene.

### SUMMARY

A process for the synthesis of fluorinated olefins of the formula CF₃CF=CHX, wherein X is F or H, comprising contacting hexafluoropropene with hydrogen chloride in the vapor phase, in the presence of a catalyst, at a temperature in the range of from 200°C to 350°C, wherein the mole ratio of hydrogen chloride to hexafluoropropene is from 2:1 to 4:1, separating the 1-chloro-1,2,3,3,3-pentafluoro-1-propene (CF₃CF=CClF, CFC-1215yb), 1,1-dichloro-2,3,3,3-tetrafluoro-1-propene (CF₃CF=CCl₂, CFC-1214ya) and hydrogen fluoride reaction products from unreacted hexafluoropropene, and hydrogen chloride by distillation, hydrogenating either the 1-chloro-1,2,3,3,3-pentafluoro-1-propene, 1,1-dichloro-2,3,3,3-tetrafluoro-1-propene or mixture thereof over a catalyst, and dehydrochlorinating the said hydrogenation product to produce either 1,2,3,3,3-pentafluoro-1-propene (CF₃CF=CHF, HFC-1225ye) or 2,3,3,3-tetrafluoro-1-propene (CF₃CF=CH₂, HFC-1234yf).

### DETAILED DESCRIPTION

A process for the synthesis of fluorinated olefins of the formula CF₃CF=CHX, wherein X is F or H, comprising contacting hexafluoropropene with hydrogen chloride in the vapor phase, in the presence of a catalyst, at a temperature in the range of from 200 °C to 350 °C, wherein the mole ratio of hydrogen chloride to hexafluoropropene is from 2:1 to 4: 1, separating the 1-chloro-1,2,3,3,3-pentafluoro-1-propene (CF₃CF=CClF, CFC-1215yb). 1,1-dichloro-2,3,3,3-tetrafluoro-1-propene (CF₃CF=CCl₂, CFC-1214ya) and hydrogen fluoride reaction products from unreacted hexafluoropropene, and hydrogen chloride by distillation, hydrogenating either the 1-chloro-1,2,3,3,3-pentafluoro-1-propene, 1,1-dichloro-2,3,3,3-tetrafluoro-1-propene or mixture thereof over a catalyst, and dehydrochlorinating the said hydrogenation product to produce either 1,2,3,3,3-pentafluoro-1-propene (CF₃CF=CHF, HFC-1225ye) or 2,3,3,3-tetrafluoro-1-propene (CF₃CF=CH₂, HFC-1234yf). After reading this specification, skilled artisans appreciate that other aspects and embodiments are possible without departing from the scope of the invention.

Other features and benefits of any one or more of the embodiments will be apparent from the following detailed description, and from the claims.

Before addressing details of embodiments described below, some terms are defined or clarifed.

As used herein, an azeotropic composition is a constant boiling liquid admixture of two or more substances wherein the admixture distills without substantial composition change and behaves as a constant boiling composition. Constant boiling compositions, which are characterized as azeotropic, exhibit either a maximum or a minimum boiling point, as compared with that of the non-azeotropic mixtures of the same substances. Azeotropic compositions as used herein include homogeneous azeotropes which are liquid admixtures of two or more substances that behave as a single substance, in that the vapor, produced by partial evaporation or distillation of the liquid, has the same composition as the liquid. Azeotropic compositions as used herein also include heterogeneous azeotropes where the liquid phase splits into two or more liquid phases. In these embodiments, at the azeotropic point, the vapor phase is in equilibrium with two liquid phases and all three phases have different compositions. If the two equilibrium liquid phases of a heterogeneous azeotrope are combined and the composition of the overall liquid phase calculated, this would be identical to the composition of the vapor phase.

For the purpose of this discussion, near-azeotropic composition means a composition that behaves like an azeotrope (i.e., has constant boiling characteristics or a tendency not to fractionate upon boiling or evaporation). Thus, the composition of the vapor formed during boiling or evaporation is the same as or substantially the same as the original liquid composition. Hence, during boiling or evaporation, the liquid composition, if it changes at all, changes only to a minimal or negligible extent. This is to be contrasted with non-azeotropic compositions in which during boiling or evaporation, the liquid composition changes to a substantial degree.

Near-azeotropic compositions exhibit dew point pressure and bubble point pressure with virtually no pressure differential. That is to say that the difference in the dew point pressure and bubble point pressure at a given temperature will be a small value. It may be stated that compositions with a difference in dew point pressure and bubble point pressure of less than or equal to 3 percent (based upon the bubble point pressure) may be considered to be a near-azeotropic.

It is also recognized that both the boiling point and the weight percentages of each component of the azeotropic or near-azeotropic liquid composition may change when the azeotropic or near-azeotropic liquid composition is subjected to boiling at different pressures. Thus, an azeotropic or a near-azeotropic composition may be defined in terms of the unique relationship that exists among the components or in terms of the compositional ranges of the components or in terms of exact weight percentages of each component of the composition characterized by a fixed boiling point at a specified pressure. It is also recognized in the art that various azeotropic compositions (including their boiling points at particular pressures) may be calculated (see, e.g., W. Schotte Ind. Eng. Chem. Process Des. Dev. (1980) 19, 432-439). Experimental identification of azeotropic compositions involving the same components may be used to confirm the accuracy of such calculations and/or to modify the calculations at the same or other temperatures and pressures.

In one embodiment, the process is one to manufacture 1-chloro-1,2,3,3,3-pentafluoro-1-propene, an intermediate useful in the manufacture of 1,2,3,3,3-pentafluoro-1-propene. 1-chloro-1,2,3,3,3-pentafluoro-1-propene as used herein refers to the isomers, *E*-1-chloro-1,2,3,3,3-pentafluoro-1-propene or Z-1-chloro-1,2,3,3,3-pentafluoro-1-propene, as well as any combinations or mixtures of such isomers. In another embodiment, the process is one to manufacture 1,1-dichloro-2,3,3,3-tetrafluoro-1-propene, an intermediate useful in the manufacture of 2,3,3,3-tetrafluoro-1-propene.

In one embodiment, 1-chloro-1,2,3,3,3-pentafluoro-1-propene is manufactured through the reaction of hexafluoropropene with hydrogen chloride in the presence of a catalyst. In one embodiment, the ratio of hydrogen chloride to hexafluoropropene is from 2:1 to 4:1. In another embodiment, the ratio of hydrogen chloride to hexafluoropropene is less than 3:1. In one embodiment, the temperature range for the reaction is from 200°C to 350°C. In one embodiment, the catalyst is selected from the group consisting of aluminum fluoride, fluorided alumina, metals on aluminum fluoride, chromium oxide, and metals on chromium oxide. In one embodiment, the catalyst is selected from the group consisting of silver on aluminum fluoride and silver on fluorided alumina. In one embodiment, the catalyst is silver on chromium oxide. In one embodiment the catalyst is selected from palladium on aluminum fluoride and palladium on fluorided alumina. In one embodiment, the catalyst is palladium on chromium oxide.

The reaction vessel is constructed from materials which are resistant to the action of hydrogen fluoride. Examples include stainless steels, high nickel alloys such as monel, "Hastelloy" and "Inconel", and plastics such as polyethylene, polypropylene, polychlorotrifluoroethylene and polytetrafluoroethylene. The high nickel alloys are preferred because of the superacidities of some fluorination catalysts in combination with liquid HF. For reactions at a temperature either below the boiling point of hydrogen fluoride (19.5 °C) or below the boiling point of the most volatile reactant, the reaction vessel can be closed or open to the atmosphere if provisions to exclude moisture are taken. For reactions at a temperature at or above the boiling point of hydrogen fluoride or the most volatile component, a closed vessel or a pressure-regulated partially open reaction is used to minimize the loss of the reactants.

In some embodiments, pressure is not critical. In other embodiments, the process is performed at atmospheric and autogenous pressures. Means can be provided for the venting of the excess pressure of hydrogen fluoride formed in the substitution reaction and can offer an advantage in minimizing the formation of side products.

In another embodiment, 1,1-dichloro-2,3,3,3-tetrafluoro-1-propene is manufactured through the reaction of hexafluoropropene with hydrogen chloride in the presence of a catalyst.

At atmospheric pressure, the boiling points of hydrofluoric acid and 1-chloro-1,2,3,3,3-pentafluoro-1-propene are 19.5 °C and +8 °C, respectively. HF may be removed from the halogenated hydrocarbon components of the product mixture using, for example, standard aqueous solution scrubbing techniques. However, the production of substantial amounts of scrubbing discharge can create aqueous waste disposal concerns.

In a second step, the 1-chloro-1,2,3,3,3-pentafluoro-1-propene, 1,1-dichloro-2,3,3,3-tetrafluoro-1-propene or mixture thereof is reacted with hydrogen in the presence of a catalyst. In one embodiment, catalysts suitable for carrying out the reaction with H₂ comprise palladium and may optionally comprise additional Group VIII metals (e.g., Pt, Ru, Rh or Ni). In one embodiment, the palladium is supported on alumina, fluorided alumina, aluminum fluoride or a mixture thereof. The palladium-containing precursor used to prepare the catalyst is preferably a palladium salt (e.g., palladium chloride). Other metals, when used, may be added to the support during the preparation of the catalyst. In another embodiment the hydrogenation catalyst can be another supported palladium catalyst, such as palladium on carbon or palladium on charcoal.

In one embodiment, the step of hydrogenation of 1-chloro-1,2,3,3,3-pentafluoro-1-propene, 1,1-dichloro-2,3,3,3-tetrafluoro-1-propene or mixtures thereof occurs simultaneously with dehydrochlorination to provide 1,2,3,3,3-pentafluoro-1-propene (HFC-1225ye) or 2,3,3,3-tetrafluoro-1-propene (HFC-1234yf) directly. Catalysts which accomplish these reactions in one step include palladium on alumina or palladium on fluorided alumina or aluminum fluoride.

The supported metal catalysts may be prepared by conventional methods known in the art such as by impregnation of the carrier with a soluble salt of the catalytic metal (e.g., palladium chloride or rhodium nitrate) as described by Satterfield of page 95 of Heterogenous Catalysis in Industrial Practice, 2nd edition (McGraw-Hill, New York, 1991). Palladium supported on alumina is available commercially. Another suitable procedure for preparing a catalyst containing palladium on fluorided alumina is described in U.S.Patent No. 4,873,381.

A catalytically effective amount of catalyst is the concentration of catalyst(s) on the support that is sufficient to carry out the catalytic reaction. For example, in one embodiment, the concentration of palladium on the support is typically in the range of from about 0.1 % to about 10% by weight based on the total weight of the catalyst. In another embodiment, the concentration of palladium on the support is typically in the range of about 0.1% to 5% by weight based on the total weight of the catalyst. In one embodiment, the concentration of the additional Group VIII metal, when used, is 3% by weight, or less, based on the total weight of the catalyst; but palladium is ordinarily at least 50% by weight based on the weight of the total metals present on the support. In another embodiment, palladium is at least 80% by weight based on the weight of the total metals present on the support.

In one embodiment, the relative amount of hydrogen fed during contact of C₃ClF₅ in a reaction zone containing the palladium-containing catalyst is from 1 mole of H₂ per mole of C₃ClF₅ to 5 moles of H₂ per mole of C₃ClF₅. In another embodiment, the relative amount of H₂ per mole of C₃ClF₅ is from 1 mole of H₂ per mole of C₃ClF₅ to 4 moles of H₂ per mole of C₃ClF₅. In yet another embodiment, the relative amount of H₂ per mole of C₃ClF₅ is from 1 mole of H₂ per mole of C₃ClF₅ to 2 moles H₂ per mole of C₃ClF₅.

In one embodiment, the reaction zone temperature for the catalytic hydrogenation of C₃ClF₅ is typically in the range of from 100°C to 400°C. In another embodiment, the reaction zone temperature is from 125°C to 350°C. In one embodiment, the contact time is typically in the range of from 1 to 450 seconds. In another embodiment, the contact time is typically from in the range of from 10 to 120 seconds. The reactions are typically conducted at near atmospheric pressure.

In one embodiment, the relative amount of hydrogen fed during contact of C₃Cl₂F₄ (CFC-1214ya) in a reaction zone containing the palladium-containing catalyst is from 1 mole of H₂ per mole of C₃Cl₂F₄ to 5 moles of H₂ per mole of C₃Cl₂F₄. In another embodiment, the relative amount of H₂ is from 1 mole of H₂ per mole of C₃Cl₂F₄ to 4 moles of H₂ per mole of C₃Cl₂F₄. In yet another embodiment, the relative amount of H₂ is from 2 moles of H₂ per mole of C₃Cl₂F₄ to 3 moles H₂ per mole of C₃Cl₂F₄.

In one embodiment, the reaction zone temperature for the catalytic hydrogenation of C₃Cl₂F₄ is typically in the range of from 100°C to 400°C. In another embodiment, the reaction zone temperature is in the range of from 125C to 350C. In one embodiment, the contact time is typically in the range of from 1 to 450 seconds. In another embodiment the reaction zone temperature is in the range of from 10 to 120 seconds. The reactions are typically conducted at near atmospheric pressure.
In another embodiment, the step of hydrogenation of 1-chloro-1,2,3,3,3-pentafluoro-1-propene, 1,1-dichloro-2,3,3,3-tetrafluoro-1-propene or mixtures thereof to produce a hydrochlorofluorocarbon, and subsequent dehydrochlorination to provide 1,2,3,3,3-pentafluoro-1-propene (HFC-1225ye) or 2,3,3,3-tetrafluoro-1-propene (HFC-1234yf) occur in separate steps with separate catalysts. One embodiment of a catalyst for such a hydrogenation is palladium on carbon or palladium on charcoal.

In another embodiment, the step of hydrogenation of 1-chloro-1,2,3,3,3-pentafluoro-1-propene, 1,1-dichloro-2,3,3,3-tetrafluoro-1-propene or mixtures thereof to produce a hydrochlorofluorocarbon, and subsequent dehydrochlorination to provide 1,2,3,3,3-pentafluoro-1-propene (HFC-1225ye) or 2,3,3,3-tetrafluoro-1-propene (HFC-1234yf) occur in separate steps with separate catalysts. One embodiment of a catalyst for such a hydrogenation is palladium on carbon or palladium on charcoal.

The subsequent dehydrochlorination of a hydrochlorofluorocarbon of the present invention may be carried out in the vapor phase. Vapor phase dehydrochlorination of a hydrochlorofluorocarbon may be suitably carried out using typical dehydrochlorination catalysts. Generally, the present dehydrochlorination may be carried out using any dehydrochlorination catalyst known in the art. These catalysts include, but are not limited to, aluminum fluoride, fluorided alumina, metals on aluminum fluoride, metals on fluorided alumina; oxides, fluorides, and oxyfluorides of magnesium, zinc and mixtures of magnesium and zinc and/or aluminum; lanthanum oxide and fluorided lanthanum oxide; chromium oxides, fluorided chromium oxides, and cubic chromium trifluoride; carbon, acid-washed carbon, activated carbon, three dimensional matrix carbonaceous materials; and metal compounds supported on carbon. The metal compounds are oxides, fluorides, and oxyfluorides of at least one metal selected from the group consisting of sodium, potassium, rubidium, cesium, yttrium, lanthanum, cerium, praseodymium, neodymium, samarium, chromium, iron, cobalt, rhodium, nickel, copper, zinc, and mixtures thereof.

Dehydrochlorination catalysts of the present invention include aluminum fluoride, fluorided alumina, metals on aluminum fluoride, and metals on fluorided alumina, as disclosed in U. S. Patent No. 5,396,000. Fluorided alumina and aluminum fluoride can be prepared as described in U. S. Patent No. 4,902,838, Suitable metals include chromium, magnesium (e.g., magnesium fluoride), Group VIIB metals (e.g., manganese), Group IIIB metals (e.g., lanthanum), and zinc. In use, such metals are normally present as halides (e.g., fluorides), as oxides and/or as oxyhalides. Metals on aluminum fluoride and metals on fluorided alumina can be prepared by procedures as described in U.S. Patent No. 4,766,260. Preferably, when supported metals are used, the total metal content of the catalyst is from 0.1 to 20 percent by weight, typically from 0.1 to 10 percent by weight. Preferred catalysts include catalysts consisting essentially of aluminum fluoride and/or fluorided alumina.

Additionally, dehydrochlorination catalysts of the present invention include oxides, fluorides, and oxyfluorides of magnesium, zinc and mixtures of magnesium and zinc and/or aluminum. A suitable catalyst may be prepared, for example by drying magnesium oxide until essentially all water is removed, e.g., for 18 hours at 100°C. The dried material is then transferred to the reactor to be used. The temperature is then gradually increased to 400°C while maintaining a flow of nitrogen through the reactor to remove any remaining traces of moisture from the magnesium oxide and the reactor. The temperature is then lowered to 200°C and a fluoriding agent, such as HF, or other vaporizable fluorine containing compounds such as HF, SF₄, CCl₃F, CCl₂F₃, CHF₃, CHClF₂ or CCl₂FCClF₂, optionally diluted with an inert gas such as nitrogen, is passed through the reactor. The inert gas or nitrogen can be gradually reduced until only HF or other vaporizable fluorine containing compounds is being passed through the reactor. At this point the temperature can be increased to 450°C and held at that temperature to convert the magnesium oxide to a fluoride content corresponding to at least 40 percent by weight, e.g., for 15 to 300 minutes, depending on the fluoriding agent flowrate and the catalyst volume. The fluorides are in the form of magnesium fluoride or magnesium oxyfluoride; the remainder of the catalyst is magnesium oxide. It is understood in the art that fluoriding conditions such as time and temperature can be adjusted to provide higher than 40 percent by weight fluoride-containing material.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Group numbers corresponding to columns within the Periodic Table of the elements use the "New Notation" convention as seen in the CRC Handbook of Chemistry and Physics, 81st Edition (2000-2001).

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below. In case of conflict, the present specification, including definitions, will control. In addition, the materials, methods, and examples are illustrative only and not intended to be limiting.

### EXAMPLES

The concepts described herein will be further described in the following examples, which do not limit the scope of the invention described in the claims.

### Catalyst preparations

### PREPARATION EXAMPLE 1

### Preparation of 95%Chromium/2%Silver Catalyst (400°C)

A solution of 784.3 g Cr(NO₃)₃[9(H₂O)] (1.96 moles) and 6.79 g AgNO₃ (0.04 mole) was prepared in 2000 mL of deionized water. The solution was treated dropwise with about 950 mL of 50/50 mix of aqueous ammonia that raised the pH to about 8.5. The slurry was stirred at room temperature overnight. The mixture was then dried in air at 110 -120°C for 48 hours. The resulting solid was then divided into two portions and one portion was calcined in air at 400°C for 24 hours. It was then pressed into discs and broken and sieved. The 12/20 mesh fraction was used in catalyst evaluation.

### PREPARATION EXAMPLE 2

### Preparation of 95%Chromium/2%Silver Catalyst (900°C)

The other half of the solid from the Preparation Example 1 above after drying at 110 -120°C was then calcined overnight at 900°C in air. It was then pressed into discs and broken and sieved. The 12/20 mesh fraction was used in catalyst evaluation.

### PREPARATION EXAMPLE 3

### Preparation of 98%Chromium/2%Palladium Catalyst (900°C)

Preparation Example 1 was repeated using solutions of chromium nitrate and palladium nitrate. The resulting solid was calcined at 900°C in air. It was then pressed into discs and broken and sieved. The 12/20 mesh fraction was used in catalyst evaluation.

### General Procedure for Catalyst Evaluation and Product Analysis

### EXAMPLES 4-6

A weighed quantity of pelletized catalyst was placed in a 5/8" (1.58 cm) diameter Inconel^{™} nickel alloy reactor tube heated in a fluidized sand bath (12/20 mesh, 15 cc). The tube was heated from 50°C to 175°C in a flow of nitrogen (50 cc/min; 8.3(10)⁻⁷m³/sec) over the course of about one hour. HF was then admitted to the reactor at a flow rate of 50 cc/min (8.3(10)⁻⁷m³/sec). After 0.5 to 2 hours the nitrogen flow was decreased to 20 cc/min (3.3(10)⁻⁷m³/sec) and the HF flow increased to 80 cc/min (1.3(10)⁻⁶m³/sec); this flow was maintained for about 1 hour. The reactor temperature was then gradually increased to 400°C over 3 to 5 hours. At the end of this period, the HF flow was stopped and the reactor cooled to 300°C under 20 sccm (3.3(10)⁻⁷m³/sec) nitrogen flow. Hexafluoropropene (HFP) and anhydrous HCl were then fed to the reactor using suitable mass controllers at the indicated temperature. All reactions were conducted at a nominal pressure of one atmosphere. The contact time for all runs in Examples 4-6 was 30 seconds. The HCl:HFP ratio was varied in Examples 4 and 5 as indicated, and was 2:1 in Example 6. The weight of catalyst used in Example 4 (Preparation Example 1) was 23.4 g; for Example 5 (Preparation Example 2) 31.6 g; and Example 6 (Preparation Example 3), 25.1 g.

Part of the total reactor effluent was sampled on-line for organic product analysis using a gas chromatograph equipped a mass selective detector (GC/MS). The gas chromatography utilized a 20 ft. (6.1 m) long x 1/8 in. (0.32 cm) diameter tube containing Krytox^{®} perfluorinated polyether on an inert carbon support. The helium flow was 30 mL/min (5.0 x 10⁻⁷ m³/sec). Gas chromatographic conditions were 60°C for an initial hold period of three minutes followed by temperature programming to 200°C at a rate of 6°C/minute. The analytical results are reported in area%. Results for the Examples are summarized in Tables 1 (for Preparation Example 1), 2 (for Preparation Example 2) and 3 (for Preparation Example 3).

### LEGEND:

HFP is hexafluoropropene
227ea is CF₃CHFCF₃
217ba is CF₃CFClCF₃
1215yb is CF₃CF=CClF
236ea is CF₃CFHCF₂H
216 is CF₃CCl₂CF₃ and CF₃CClFCF₂Cl
226ea is CF₃CFHCF₂Cl
1224 is C₃HClF₄ isomers
1214ya is CF₃CF=CCl₂

**Table 1**

| Time (hrs) | Temp | HCI:HFP | HFP | 227ea | 217ba | 1215yb | 236ea | 216 | 1214ya |
|---|---|---|---|---|---|---|---|---|---|
| 1 | 100 | 2 | 94.62 | 0.64 | 0.03 | 2.51 | 1.16 | .65 | 0.21 |
| 2 | 100 | 2 | 94.36 | 0.53 | 0 | 3.32 | 1.13 | .23 | 0.34 |
| 3 | 100 | 3 | 94.36 | 0.45 | 0 | 3.50 | 1.10 | .17 | 0.34 |
| 4 | 100 | 3 | 88.63 | 0.53 | 0 | 7.92 | 1.23 | .34 | 1.03 |
| 5 | 100 | 2 | 90.62 | 0.47 | 0 | 6.79 | 1.00 | .16 | 0.83 |
| 6 | 100 | 2 | 82.37 | 0.66 | 0.01 | 12.89 | 1.23 | .39 | 2.04 |

**Table 2**

| Temp | HCl:HFP | HFP | 1215yb | 1214ya |
|---|---|---|---|---|
| 200 | 2 | 91.14 | 7.78 | 0.79 |
| 200 | 2 | 91.45 | 7.54 | 0.79 |
| 225 | 2 | 86.13 | 11.81 | 1.75 |
| 250 | 2 | 78.43 | 16.70 | 3.45 |
| 275 | 2 | 64.38 | 27.15 | 7.80 |
| 300 | 2 | 57.15 | 30.92 | 11.16 |
| 300 | 3 | 66.37 | 25.54 | 7.41 |
| 350 | 2 | 43.50 | 33.49 | 21.89 |
| 350 | 6 | 58.02 | 27.25 | 13.12 |
| 100 | 2 | 99.99 | 0 | 0 |
| 125 | 2 | 99.99 | 0 | 0 |
| 150 | 2 | 99.99 | 0 | 0 |
| 175 | 2 | 99.97 | 0 | 0 |
| 200 | 2 | 99.9 | 0 | 0 |

**Table 3**

| Temp | HCI:HFP | HFP | 227ea | 133a | 1215yb | 1224 | 226ea | 1214ya |
|---|---|---|---|---|---|---|---|---|
| 100 | 2 | 99.77 | 0 | 0 | 0.09 | 0 | 0.03 | 0.01 |
| 125 | 2 | 99.31 | 0 | 0 | 0.51 | 0 | 0.03 | 0.02 |
| 150 | 2 | 98.66 | 0.06 | 0 | 1.09 | 0 | 0.04 | 0.05 |
| 200 | 2 | 99.59 | 0 | 0 | 0.18 | 0 | 0.01 | 0.01 |
| 225 | 2 | 98.77 | 0 | 0 | 0.99 | 0 | 0.01 | 0.03 |
| 250 | 2 | 87.64 | 0.11 | 0 | 10.45 | 0 | 0.11 | 1.46 |
| 275 | 2 | 50.84 | 0.27 | 0.02 | 27.52 | 0 | 0.57 | 20.25 |
| 300 | 2 | 32.75 | 0.40 | 0.05 | 28.12 | 0 | 0.94 | 36.92 |
| 300 | 2 | 30.07 | 0.51 | 0.04 | 27.22 | 0.09 | 1.20 | 40.29 |
| 325 | 2 | 22.73 | 0.50 | 0.04 | 24.19 | 0 | 1.21 | 50.25 |
| 350 | 2 | 22.32 | 0.40 | 0.05 | 24.32 | 0 | 0.96 | 50.14 |
| 375 | 2 | 12.93 | 0.50 | 0.04 | 21.05 | 0.57 | 1.45 | 59.90 |
| 400 | 2 | 9.4 | 0.59 | 0.02 | 19.73 | 2.10 | 1.81 | 59.21 |

### EXAMPLES 7-8

Legend
CFC-1214ya is CF₃CF=CCl₂
E/Z-CFC-1215yb is CF₃CF=CClF
HFP is CF₃CF=CF₂
HCFC-1224 is C₃HClF₄
E/Z-HFC-1225ye is CF₃CF=CHF
HFC-1234yf is CF₃CF=CH₂
HFC-236ea is CF₃CHFCHF₂
HCFC-244eb is CF₃CHFCH₂Cl
HFC-245eb is CF₃CHFCH₂F
HFC-254eb is CF₃CHFCH₃
HFC-263fb is CF₃CH₂CH₃

### EXAMPLE 7

### Hydrodechlorination of CFC-1215yb (CF₃CF=CClF)

A commercial palladium on aluminum oxide catalyst (0.5% Pd/Al₂O₃, 10 cc, 14.45 g, 12-20 mesh (1.68-0.84 mm)) was placed in a 30.5 cm x 1.27 cm o.d. Hastelloy® tube. The tube was connected to a reactor system and surrounded with an electrically-heated furnace. The catalyst was first dried for three hours under a nitrogen purge (25 sccm, 4.2x10⁻⁷ m³/s) as the temperature of the furnace was raised to 300°C. The reactor was allowed to cool to 150°C, and then hydrogen gas (20 sccm, 3.3x10⁻⁷ m³/s) was passed into the reactor for three hours as the temperature in the reactor was increased to 300°C. The reactor was cooled again to 150°C under a flow of nitrogen (20 sccm, 3.3x10⁻⁷ m³/s). The catalyst was then fluorinated with a mixture of nitrogen and hydrogen fluoride according to following sequence (time in hours, flow rate nitrogen, flow rate HF, temperature):
2 h, 7.5x10⁻⁷ m³/s, 8.3x10⁻⁸ m³/s, 150°C; 2h, 6.6x10⁻⁷ m³/s, 1.7x10⁻⁷ m³/s, 150°C; 2 h, 6.6x10⁻⁷ m³/s, 1.7x10⁻⁷ m³/s, 200°C; 2 h, 6.6x10⁻⁷ m³/s, 1.7x10⁻⁷ m³/s, 250°C; 2 h, 4.2x10⁻⁷ m³/s, 4.2x10⁻⁷ m³/s, 250°C. The flow of hydrogen fluoride was then stopped and the reactor was purged with nitrogen.

This catalyst, after use in several other hydrodechlorination reactions, was activated with air. Nitrogen was passed through the reactor (50 sccm, 8.3x10⁻⁷ m³/s) as the temperature of the furnace was raised to 300°C. Air was then co-fed to the reactor along with nitrogen at 300°C. The percentage of air in the air/nitrogen mixture was increased from 5.3 volume % to 100 volume % in eight increments over the course of two hours. After passing 100% air through the reactor for one hour, the reactor was purged with nitrogen (75 sccm, 1.25x10⁻⁶ m³/s). The catalyst was then reduced under a flow of hydrogen (50 sccm, 8.3x10⁻⁷ m³/s) at 300°C for two hours. The reactor was then allowed to cool to 200°C under a hydrogen flow.

A mixture of hydrogen, CFC-1215yb (0.4:1 E:Z mixture), and nitrogen in a 1:1:3 molar ratio was then passed through the catalyst bed at 200°C with a contact time of 30 seconds. The pressure in the reactor was nominally atmospheric. The composition of the organic components in the reactor effluent as determined by GC-MS (and corroborated by 1 Hand 19F NMR) is given in Table 4 below.

**Table 4**

| | |
|---|---|
| E/Z-CFC-1215yb | 12.5 mole % (E/Z ratio =0_{.}5:1) |
| HFC-1234yf | 1.7 mole % |
| HFP | 8.0 mole % |
| Mixture of Z-HFC1225ye and HFC-254eb | 24.6 mole %* |
| E-HFC-1225ye | 32.0 mole % |
| HFC-236ea | 0.5 mole % |
| HFC-245eb | 19.5 mole % |
| HCFC-244eb | 0.9 mole % |

| | |
|---|---|
| * This mixture is primarily (about 89%) Z-1225ye. | |

### EXAMPLE 8

### Hydrodechlorination of CFC-1214ya (CF₃CF=CCl₂)

The catalyst from the previous example was pre-treated with air followed by hydrogen as outlined above.

A mixture of hydrogen, CFC-1214ya, and nitrogen in a 1:1:9 molar ratio was then passed through the catalyst bed at 200°C with a contact time of 14 seconds. The pressure in the reactor was nominally atmospheric. The composition of the organic components in the reactor effluent as determined by GC-MS is given in Table 5 below. Minor amounts of four carbon products (<1%) and HFC-245cb were also detected.

**Table 5**

| | |
|---|---|
| CFC-1214ya | 52.4 mole % |
| HFC-1234yf | 33.8 mole % |
| HFC-263fb | 2.9 mole % |
| HFC-254eb | 7.1 mole % |
| HCFC-1224 | 2.8 mole % |

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims bellow. Accordingly, the specification and figures are to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination. Further, reference to values stated in ranges include each and every value within that range.

## Claims

1. A process for the synthesis of fluorinated olefins of the formula CF₃CF=CHX, wherein X is F or H comprising:
a) contacting hexafluoropropene with hydrogen chloride in the vapor phase, in the presence of a catalyst, at a temperature in the range from 200 °C to 350 °C, to produce a mixture comprising 1-chloro-1,2,3,3,3-pentafluoro-1-propene, 1,1-dichloro-2,3,3,3-tetrafluoro-1-propene and hydrogen fluoride;
b) separating the 1-chloro-1 ,2,3,3,3.pentafluoro.1-propene, 1,1-dichloro-2,3,3,3-tetrafluoro-1-propene and optionally hydrogen fluoride products from unreacted hexafluoropropene, hydrogen chloride and hydrogen fluoride by distillation;
c) hydrogenating either the 1-chloro-1,2,3,3,3-pentafiuoro-1-propene, 1,1-dichloro-2,3,3,3-tetrafluoro-1-propene or mixtures thereof over a catalysts to produce a hydrogenation product, and
d) dehydrochlorinating the said hydrogenation product over a catalyst to produce either 1225ye, 1234yf or mixtures thereof.

2. The process of claim 1 wherein the hydrogenation and dehydrochlorination are performed simultaneously over a catalyst of palladium on aluminum fluorided or palladium on fluorided alumina.

3. The process of claim 1 wherein the catalyst for the reaction of hexafluoropropene with hydrogen chloride is selected from the group consisting of aluminium fluoride, fluorided alumina, metals on aluminum fluoride chromium oxide, and metals on chromium oxide.

4. The process of claim 1 wherein the catalyst for the reaction of hexafluoropropene with hydrogen chloride is selected from the group consisting of silver on aluminum fluoride and silver on fluorided alumina.

5. The process of claim 1 wherein the catalyst for the reaction of hexafluoropropene with hydrogen chloride is silver on chromium oxide.

6. The process of claim 1 wherein the ratio of hydrogen chloride to hexafluoropropene is from 2:1 to 4:1.

7. The process of claim 1 wherein the ratio of hydrogen chloride to hexafluoropropene is less than 3:1.

8. The process of claim 1 wherein the catalyst for the hydrogenation is palladium on carbon.

9. The process of claim 8 wherein the catalyst further comprises one or more of the group consisting of platinum, ruthenium, rhodium or nickel.

10. The process of claim 1, wherein the catalyst for dehydrochlorinating the hydrogenation product is selected from the group consisting of aluminum fluoride, fluorided alumina, metals on aluminum fluoride, metals on fluorided alumina; oxides, fluorides, and oxyfluorides of magnesium, zinc and mixtures of magnesium and zinc and/or aluminum; lanthanum oxide and fluorided lanthanum oxide; chromium oxides, fluorided chromium oxides, and cubic chromium trifluoride; carbon, acid-washed carbon, activated carbon, three dimensional matrix carbonaceous materials; and metal compounds supported on carbon.

11. A process for the synthesis of 1-chloro-1,2,3,3,3-pentafluoro-1-propene, 1,1-dichloro-2,3,3,3-tetrafluoro-1-propene or a mixture thereof, comprising:
a) contacting hexafluoropropene with hydrogen chloride in the vapor phase, in the presence of a catalyst, at a temperature in the range from 200 °C to 350 °C, to produce a mixture comprising 1-chloro-1,2,3,3,3-pentafluoro-1-propene, 1,1-dichloro-2,3,3,3-tetrafluoro-1-propene;
b) separating the 1-chloro-1,2,3,3,3-pentafluoro-1-propene, 1,1-dichloro-2,3,3,3-tetrafluoro-1-propene and optionally hydrogen fluoride products from unreacted hexafluoropropene, hydrogen chloride and hydrogen fluoride by distillation;

12. The process of claim 11 wherein the catalyst for the reaction of hexafluoropropene with hydrogen chloride is selected from the group consisting of aluminum fluoride, fluorided alumina, metals on aluminum fluoride chromium oxide, and metals on chromium oxide.

13. The process of claim 11 wherein the catalyst for the reaction of hexafluoropropene with hydrogen chloride is selected from the group consisting of silver on aluminum fluoride and silver on fluorided alumina.

14. The process of claim 11 wherein the catalyst for the reaction of hexafluoropropene with hydrogen chloride is silver on chromium oxide.

15. The process of claim 11 wherein the ratio of hydrogen chloride to hexafluoropropene is from 2:1 to 4:1.

## Patentansprüche

1. Verfahren zur Synthese von fluorierten Olefinen mit der Formel CF₃CF=CHX, wobei X für F oder H steht, wobei das Verfahren aufweist:
a) Inkontaktbringen von Hexafluorpropen mit Chlorwasserstoff in der Dampfphase in Anwesenheit eines Katalysators bei einer Temperatur im Bereich von 200°C bis 350°C, um ein Gemisch zu erzeugen, das 1-Chlor-1,2,3,3,3-pentafluor-1-propen, 1,1-Dichlor-2,3,3,3-tetrafluor-1-propen und Fluorwasserstoff aufweist;
b) Trennen des 1-Chlor-1,2,3,3,3-pentafluor-1-propens, 1,1-Dichlor-2,3,3,3-tetrafluor-1-propens und wahlweise der Fluorwasserstoffprodukte von nicht umgesetztem Hexafluorpropen, Chlorwasserstoff und Fluorwasserstoff durch Destillation;
c) Hydrieren entweder des 1-Chlor-1,2,3,3,3-pentafluor-1-propens, des 1,1-Dichlor-2,3,3,3-tetrafluor-1-propens oder von Gemischen davon über einem Katalysator, um ein Hydrierungsprodukt zu erzeugen, und
d) Dehydrochlorieren des Hydrierungsprodukts über einem Katalysator, um entweder 1225ye, 1234yf oder Gemische davon zu erzeugen.

2. Verfahren nach Anspruch 1, wobei die Hydrierung und Dehydrochlorierung gleichzeitig über einem Katalysator aus Palladium auf Aluminiumfluorid oder Palladium auf fluoridiertem Aluminiumoxid durchgeführt werden.

3. Verfahren nach Anspruch 1, wobei der Katalysator für die Reaktion von Hexafluorpropen mit Chlorwasserstoff aus der Gruppe ausgewählt ist, die aus Aluminiumfluorid, fluoridiertem Aluminiumoxid, Metallen auf Aluminiumfluorid/Chromoxid und Metallen auf Chromoxid besteht.

4. Verfahren nach Anspruch 1, wobei der Katalysator für die Reaktion von Hexafluorpropen mit Chlorwasserstoff aus der Gruppe ausgewählt ist, die aus Silber auf Aluminiumfluorid und Silber auf fluoridiertem Aluminiumoxid besteht.

5. Verfahren nach Anspruch 1, wobei der Katalysator für die Reaktion von Hexafluorpropen mit Chlorwasserstoff Silber auf Chromoxid ist.

6. Verfahren nach Anspruch 1, wobei das Verhältnis von Chlorwasserstoff zu Hexafluorpropen gleich 2:1 bis 4:1 ist.

7. Verfahren nach Anspruch 1, wobei das Verhältnis von Chlorwasserstoff zu Hexafluorpropen kleiner als 3:1 ist.

8. Verfahren nach Anspruch 1, wobei der Katalysator für die Hydrierung Palladium auf Kohlenstoff ist.

9. Verfahren nach Anspruch 8, wobei der Katalysator ferner ein oder mehrere Elemente aus der Gruppe aufweist, die aus Platin, Ruthenium, Rhodium oder Nickel besteht.

10. Verfahren nach Anspruch 1, wobei der Katalysator für die Dehydrochlorierung des Hydrierungsprodukts aus der Gruppe ausgewählt ist, die aus Aluminiumfluorid, fluoridiertem Aluminiumoxid, Metallen auf Aluminiumfluorid, Metallen auf fluoridiertem Aluminiumoxid, Oxiden, Fluoriden und Oxyfluoriden von Magnesium, Zink und Gemischen von Magnesium und Zink und/oder Aluminium, Lanthanoxid und fluoridiertem Lanthanoxid; Chromoxiden; fluoridierten Chromoxiden und kubischem Chromtrifluorid; Kohlenstoff, säuregewaschenern Kohlenstoff, Aktivkohle, kohlenstoffhaltigen Materialien mit dreidimensionaler Matrix und Metallverbindungen auf Kohlenstoff als Träger besteht.

11. Verfahren zur Synthese von 1-Chlor-1,2,3,3,3-pentafluor-1-propen, 1,1-Dichlor-2,3,3,3-tetrafluor-1-propen oder eines Gemischs davon, wobei das Verfahren aufweist:
a) Inkontaktbringen von Hexafluorpropen mit Chlorwasserstoff in der Dampfphase in Anwesenheit eines Katalysators bei einer Temperatur im Bereich von 200°C bis 350°C, um ein Gemisch zu erzeugen, das 1-Chlor-1,2,3,3,3-pentafluor-1-propen und 1,1-Dichlor-2,3,3,3-tetrafluor-1-propen aufweist;
b) Trennen des 1-Chlor-1,2,3,3,3-pentafluor-1-propens, 1,1-Dichlor-2,3,3,3-tetrafluor-1-propens und wahlweise von Fluorwasserstoffprodukten von nicht umgesetztem Hexafluorpropen, Chlorwasserstoff und Fluorwasserstoff durch Destillation.

12. Verfahren nach Anspruch 11, wobei der Katalysator für die Reaktion von Hexafluorpropen mit Chlorwasserstoff aus der Gruppe ausgewählt ist, die aus Aluminiumfluorid, fluoridiertem Aluminiumoxid, Metallen auf Aluminiumfluorid/Chromoxid und Metallen auf Chromoxid besteht.

13. Verfahren nach Anspruch 11, wobei der Katalysator für die Reaktion von Hexafluorpropen mit Chlorwasserstoff aus der Gruppe ausgewählt ist, die aus Silber auf Aluminiumfluorid und Silber auf fluoridiertem Aluminiumoxid besteht.

14. Verfahren nach Anspruch 11, wobei der Katalysator für die Reaktion von Hexafluorpropen mit Chlorwasserstoff Silber auf Chromoxid ist.

15. Verfahren nach Anspruch 11, wobei das Verhältnis von Chlorwasserstoff zu Hexafluorpropen gleich 2:1 bis 4:1 ist.

## Revendications

1. Procédé de synthèse d'oléfines fluorées de la formule CF₃CF=CHX, où X est F ou H, comprenant:
a) mettre de l'hexafluoropropène en contact avec du chlorure d'hydrogène en phase vapeur, en présence d'un catalyseur, à une température dans la plage de 200°C à 350°C, pour produire un mélange comprenant du 1-chloro-1,2,3,3,3-pentafluoro-1-propène, du 1,1-dichloro-2,3,3,3-tétrafluoro-1-propène et du fluorure d'hydrogène;
b) séparer le 1-chloro-1,2,3,3,3-pentafluoro-1-propène, le 1,1-dichloro-2,3,3,3-tétrafluoro-1-propène et optionnellement les produits de fluorure d'hydrogène de l'hexafluropropène, du chlorure d'hydrogène et du fluorure d'hydrogène inaltérés par distillation;
c) hydrogéner soit le 1-chloro-1,2,3,3,3-pentafluoro-1-propène soit le 1,1-dichloro-2,3,3,3-tétrafluoro-1-propène ou des mélanges de ceux-ci sur un catalyseur pour produire un produit d'hydrogénation, et
d) déshydrochlorer ledit produit d'hydrogénation sur un catalyseur pour produire soit du 1225ye soit du 1234fy ou des mélanges de ceux-ci.

2. Procédé selon la revendication 1, dans lequel l'hydrogénation et la déshydrochloration sont effectuées simultanément sur un catalyseur de palladium sur fluorure d'aluminium ou de palladium sur alumine fluorée.

3. Procédé selon la revendication 1, dans lequel le catalyseur pour la réaction de l'hexafluoropropène avec du chlorure d'hydrogène, est sélectionné parmi le groupe consistant en fluorure d'aluminium, alumine fluorée, métaux sur fluorure d'aluminium/oxyde de chrome et métaux sur oxyde de chrome.

4. Procédé selon la revendication 1, dans lequel le catalyseur pour la réaction de l'hexafluoropropène avec du chlorure d'hydrogène, est sélectionné parmi le groupe consistant en argent sur fluorure d'aluminium et argent sur alumine fluorée.

5. Procédé selon la revendication 1, dans lequel le catalyseur pour la réaction de l'hexafluoropropène avec du chlorure d'hydrogène est de l'argent sur oxyde de chrome.

6. Procédé selon la revendication 1, dans lequel le rapport de chlorure d'hydrogène à hexafluoropropène est de 2:1 à 4:1.

7. Procédé selon la revendication 1, dans lequel le rapport de chlorure d'hydrogène à hexafluoropropène est de moins de 3:1.

8. Procédé selon la revendication 1, dans lequel le catalyseur pour l'hydrogénation est du palladium sur carbone.

9. Procédé selon la revendication 8, dans lequel le catalyseur comprend en plus un ou plusieurs du groupe consistant en platine, ruthénium, rhodium ou nickel.

10. Procédé selon la revendication 1, dans lequel le catalyseur pour déshydrochlorer le produit d'hydrogénation est sélectionné parmi le groupe consistant en fluorure d'aluminium, alumine fluorée, métaux sur fluorure d'aluminium, métaux sur alumine fluorée; oxydes, fluorures et oxyfluorures de magnésium, zinc et mélanges de magnésium et zinc et/ou d'aluminium; oxyde de lanthane et oxyde de lanthane fluoré; oxydes de chrome, oxydes de chrome fluorés et trifluorure de chrome cubique; carbone, charbon lavé à l'acide, charbon actif, substances carbonées à matrice tridimensionnelle et composés métalliques supportés sur du carbone.

11. Procédé de synthèse de 1-chloro-1,2,3,3,3-pentafluoro-1-propène, de 1,1-dichloro-2,3,3,3-tétrafluoro-1-propène ou d'un mélange de ceux-ci, comprenant:
a) mettre de l'hexafluoropropène en contact avec du chlorure d'hydrogène en phase vapeur, en présence d'un catalyseur, à une température dans la plage de 200°C à 350°C, pour produire un mélange comprenant du 1-chloro-1,2,3,3,3-pentafluoro-1-propène, du 1,1-dichloro-2,3,3,3-tétrafluoro-1-propène;
b) séparer le 1-chloro-1,2,3,3,3-pentafluoro-1-propène, le 1,1-dichloro-2,3,3,3-tétrafluoro-1-propène et optionnellement les produits de fluorure d'hydrogène de l'hexafluropropène, du chlorure d'hydrogène et du fluorure d'hydrogène inaltérés par distillation.

12. Procédé selon la revendication 11, dans lequel le catalyseur pour la réaction de l'hexafluoropropène avec du chlorure d'hydrogène est sélectionné parmi le groupe consistant en fluorure d'aluminium, alumine fluorée, métaux sur fluorure d'aluminium/oxyde de chrome et métaux sur oxyde de chrome.

13. Procédé selon la revendication 11, dans lequel le catalyseur pour la réaction de l'hexafluoropropène avec du chlorure d'hydrogène est sélectionné parmi le groupe consistant en argent sur fluorure d'aluminium et argent sur alumine fluorée.

14. Procédé selon la revendication 11, dans lequel le catalyseur pour la réaction de l'hexafluoropropène avec du chlorure d'hydrogène est de l'argent sur oxyde de chrome.

15. Procédé selon la revendication 11, dans lequel le rapport de chlorure d'hydrogène à hexafluoropropène est de 2:1 à 4:1.
